# EUROPÄISCHE PATENTANMELDUNG

(11) **EP 2 745 791 A2**
(43) Veröffentlichungstag der Anmeldung: **25.06.2014**
(21) Anmeldenummer: 13198357.9
(22) Anmeldetag: 19.12.2013
(51) Int. Cl.: A61B 17/86, A61B 19/02

(54) **Medizinische Sterilverpackung und Verwendung einer medizinischen Sterilverpackung**

(30) Priorität: 21.12.2012 DE 102012112945
(71) Anmelder: AESCULAP AG, 78532 Tuttlingen (DE)
(72) Erfinder: Lindner, Stephan, 78573 Wurmlingen (DE); Hoefer, Fabian, 78532 Tuttlingen (DE)
(74) Vertreter: Hoeger, Stellrecht & Partner Patentanwälte

(57) **Zusammenfassung**

Um eine medizinische Sterilverpackung, welche einen Aufnahmeraum für mindestens ein Implantat, welches ausgebildet ist zum temporären oder dauerhaften Einsetzen in einen menschlichen oder tierischen Körper, definiert, welcher Aufnahmeraum in einer Lagerstellung der Sterilverpackung steril und keimdicht verschlossen ist zum sterilen Verpacken des mindestens einen Implantats, so zu verbessern, dass Implantate steril bereitgestellt und gegebenenfalls bei Nichtgebrauch während eines chirurgischen Eingriffs einfach und sicher wieder für einen zukünftigen Eingriff aufbereitet werden können, wird vorgeschlagen, dass die Sterilverpackung aus einem oder mehreren reinig- und sterilisierbaren Materialien hergestellt ist.

## Beschreibung

Die vorliegende Erfindung betrifft eine medizinische Sterilverpackung, welche einen Aufnahmeraum für mindestens ein Implantat, welches ausgebildet ist zum temporären oder dauerhaften Einsetzen in einen menschlichen oder tierischen Körper, definiert, welcher Aufnahmeraum in einer Lagerstellung der Sterilverpackung steril und keimdicht verschlossen ist zum sterilen Verpacken des mindestens einen Implantats.

Zur Bereitstellung von Implantaten werden häufig Kunststofflagerungen für diese verwendet. Derartige Lagerungen gestatten es, die Implantate zu halten und ermöglichen dem Operateur einen wahlfreien Zugriff auf das für einen chirurgischen Eingriff jeweils benötigte Implantat. Beispiele für komplexe Implantate sind insbesondere Schrauben, beispielsweise Pedikelschrauben. Ein Problem insbesondere bei komplexen Implantaten ist es, dass für einen chirurgischen Eingriff bereitgestellte sterile Implantate, die nicht implantiert werden, im Verlauf des Eingriffs trotzdem verschmutzen können. Typische Verschmutzungen entstehen dabei beispielsweise durch Blut, Körpergewebe oder Körperflüssigkeiten, mit denen die Implantate in Kontakt kommen können. Damit die nicht benötigten Implantate nicht entsorgt werden müssen, sondern für einen zukünftigen Eingriff wieder genutzt, das heißt bereitgestellt, werden können, ist es erforderlich, die verschmutzten, nicht implantierten Implantate wieder aufzubereiten. Eine Aufbereitung in diesem Sinne umfasst insbesondere die mechanische Reinigung, beispielsweise von Hand oder in einer dafür vorgesehenen Wasch- oder Reinigungsmaschine, insbesondere auch unter Einsatz von geeigneten Reinigungs- und/oder Lösungsmitteln. Nach der Reinigung werden die Implantate dann noch sterilisiert. Dabei kommt insbesondere die in Krankenhäusern und Praxen häufig angewandte Heißdampfsterilisation zum Einsatz.

Ein Problem bei der Aufbereitung komplexer Implantate, also beispielsweise Implantaten mit Hinterschnitten oder komplizierten Gewinden, ist, dass insbesondere bei den derzeit zur Verfügung stehenden Kunststofflagerungen beziehungsweise -halterungen eine den Vorschriften entsprechende Reinigung nicht gewährleistet werden kann. Insbesondere können in unerwünschter Weise nicht nur Verunreinigungen, sondern auch Reinigungssubstanzen in Spalten und Hohlräumen der Implantate verbleiben.

Es ist daher eine Aufgabe der vorliegenden Erfindung, Implantate steril bereitstellen und gegebenenfalls bei Nichtgebrauch während eines chirurgischen Eingriffs einfach und sicher wieder für einen zukünftigen Eingriff aufbereiten zu können.

Diese Aufgabe wird bei einer medizinischen Sterilverpackung der eingangs beschriebenen Art erfindungsgemäß dadurch gelöst, dass die Sterilverpackung aus einem oder mehreren reinig- und sterilisierbaren Materialien hergestellt ist.

Die erfindungsgemäß vorgeschlagene Sterilverpackung ermöglicht es, ein vorschriftsgemäß steriles Implantat für einen chirurgischen Eingriff bereit zu stellen. Die aus einem oder mehreren reinig- und sterilisierbaren Materialien hergestellte Sterilverpackung kann also insgesamt sterilisiert werden. Dies bedeutet, dass das Implantat in der eine Schutzhülle bildenden Sterilverpackung, die wiederum selbst sterilisiert ist, für einen chirurgischen Eingriff bereitgestellt werden kann. Das Implantat wird nur dann aus der nicht nur innen, sondern auch außen sterilen medizinischen Sterilverpackung entnommen, wenn es tatsächlich für einen Eingriff benötigt wird. Nach Abschluss des chirurgischen Eingriffs werden alle nicht benötigten Implantate, die noch in ihrer jeweiligen medizinischen Sterilverpackung steril verpackt sind, in ihrer Sterilverpackung, also in ihrer Schutzhülle, wieder aufbereitet. Dies bedeutet, dass die Sterilverpackung mit dem darin enthaltenen Implantat zunächst von außen gereinigt, beispielsweise manuell oder mittels einer dafür geeigneten Wasch- oder Reinigungsmaschine, insbesondere auch unter Einsatz von Reinigungs- und Lösungsmitteln, und anschließend sterilisiert wird, beispielsweise mittels Heißdampfsterilisation. Die so gereinigten und sterilisierten Sterilverpackungen können dann beispielsweise in einem Sterilbehälter steril und keimfrei bis zum nächsten chirurgischen Eingriff gelagert werden. Insbesondere durch einfache Geometrien der Sterilverpackung kann die vorschriftsgemäße Reinigung und Sterilisierung der Sterilverpackung sichergestellt werden. Anders als bei der herkömmlichen Vorgehensweise muss also nicht das Implantat selbst gereinigt und sterilisiert werden, sondern nur die das Implantat umgebende Sterilverpackung. Insbesondere kann eine einfache Geometrie erreicht werden, indem an der Sterilverpackung auf jegliche Hinterschnitte verzichtet wird, in welchen sich Verschmutzungen und Keime ansammeln können. Hinterschnittfreie Sterilverpackungen können einfach und sicher gereinigt und sterilisiert werden. Die reinig- und sterilisierbaren Materialien, aus denen die Sterilverpackung hergestellt ist, ermöglichen vorzugsweise die Durchführung von mehreren Hundert oder Tausend Wiederaufbereitungszyklen. Dadurch lässt sich der Wiederaufbereitungsaufwand nicht nur minimieren, sondern auch die Zahl der insgesamt, beispielsweise in einem Krankenhaus oder einer Praxis, verfügbar zu haltenden Implantate minimiert werden. Auf diese Weise lassen sich signifikant Kosten senken und gleichzeitig trotzdem hohe Standards bei der Sterilqualität einhalten. Die Erfindungsgemäß vorgeschlagene Sterilverpackung ermöglicht insgesamt einen schnellen und sicheren Zugriff auf die benötigten Implantate. Im Vergleich zu denkbaren Doppel-Blister-Verpackungen entsteht verhältnismäßig wenig Müll. Der Platzbedarf für die Implantate ist gegenüber herkömmlichen Halterungen nicht vergrößert. Und schließlich können die Implantate wie gewohnt aufbereitet und gegebenenfalls auch maschinell gereinigt werden, allerdings nicht lose, sondern eben in der Sterilverpackung. Vorzugsweise ist die Sterilverpackung gasdicht, insbesondere luftdicht und/oder dampfdicht. Eine Leckrate ist günstigerweise kleiner als etwa 10⁻³ mbar/s. Insbesondere ist die vorgeschlagene Sterilverpackung einfach und praktisch handhabbar, lagerfähig und vorzugsweise bakteriendicht.

Günstig ist es, wenn die Materialien lösungsmittelbeständig sind gegen Reinigungs- und Lösungsmittel mit einem pH-Wert in einem Bereich von etwa 2 bis etwa 12. Vorzugsweise sind das oder die Materialien lösungsmittelbeständig gegen Reinigungs- und Lösungsmittel mit einem pH-Wert in einem Bereich von etwa 3 bis etwa 11. Eine Reinigung kann so wahlweise in einem sauren oder basischen Milieu erfolgen, ohne dass die Sterilverpackung durch die Reinigung beschädigt und dadurch die Sterilität des in der Sterilverpackung verpackten Implantats gefährdet wird. Vorzugsweise sind die Materialien, aus denen die Sterilverpackung hergestellt ist, gegen Reinigungsmittel resistent, die sauer, neutral oder alkalisch sind und optional einen Tensidzusatz enthalten können. Ferner sind die Materialien günstigerweise mechanisch stabil, insbesondere gegen Abwischen mit einem Tuch und/oder gegen eine Tauchdesinfektion und Reinigung mit einer Bürste sowie optional auch gegen eine Reinigung mit Ultraschall. Eine Reinigungstemperatur, insbesondere der verwendeten Reinigungsmittel, liegt vorzugsweise in einem Bereich von etwa 40°C bis etwa 98°C. Eine Reinigungszeit liegt günstigerweise in einem Bereich von etwa 1 Minute bis etwa 60 Minuten.

Um insbesondere die gereinigte Sterilverpackung mittels Heißdampfsterilisation sterilisieren zu können, ist es vorteilhaft wenn das oder die Materialien wärme-/hitzebeständig sind bis zu einer Temperatur von mindestens etwa 150°C. Insbesondere ist es günstig, wenn das oder die Materialien wärme-/ hitzebeständig sind bis zu einer Temperatur von mindestens etwa 130°C. Mit derartigen Materialien ist es möglich, die Sterilverpackung in herkömmlichen Heißdampfsterilisationsgeräten zu sterilisieren, und zwar ohne dass die Sterilverpackung beschädigt und die Sterilität des in ihr verpackten Implantats gefährdet wird. Vorzugsweise ist die Sterilverpackung derart ausgebildet, dass eine Sterilisation mit Wasserdampf möglich wird, insbesondere bei einem Druck in einem Bereich von etwa 1 bar bis etwa 4 bar, einer Temperatur von etwa 100°C bis etwa 144°C und bei einer Sterilisationsdauer in einem Bereich von etwa 1 Minute bis etwa 60 Minuten.

Vorzugsweise sind das oder die Materialien druckbeständig bei Drücken bis mindestens etwa 5 bar. Vorteilhafterweise sind das oder die Materialien druckbeständig bei Drücken bis mindestens etwa 3 bar. Vorzugsweise beträgt die Druckbeständigkeit oder Druckfestigkeit mindestens 7 bar. Mit derartigen Materialien ist es möglich, eine Sterilverpackung auszubilden, welche in herkömmlichen Heißdampfsterilisationsgeräten einer Heißdampfsterilisation unterzogen werden kann, ohne dass die Sterilverpackung geschädigt wird.

Für die Handhabung der Sterilverpackung ist es vorteilhaft, wenn diese formstabil ist. Insbesondere ist sie derart ausgebildet, dass sie bei Temperaturen von mindestens etwa 150°C sowie bei Drücken bis mindestens etwa 5 bar ihre ursprüngliche Form beibehält oder im Wesentlichen beibehält. Dadurch kann insbesondere verhindert werden, dass sich im Falle einer Verformung der Sterilverpackung Hinterschnitte oder komplexe Geometrien an dieser ausbilden können, welche nicht oder nur mit einem extrem großen Reinigungsaufwand gereinigt werden können.

Um einem Anwender die Nutzung der Sterilverpackung zu erleichtern, ist es vorteilhaft, wenn das oder die Materialien transparent oder transluzent sind. So kann ein Anwender beispielsweise direkt erkennen, was in der Sterilverpackung enthalten ist. Insbesondere kann so das in der Sterilverpackung verpackte Implantat von einem Anwender auf Sicht ausgewählt werden.

Günstigerweise sind das oder die Materialien Glas, Metall, Kunststoff oder Keramik. Insbesondere kann die Sterilverpackung aus mehreren unterschiedlichen Materialien hergestellt werden, beispielsweise aus beliebigen Kombinationen der genannten Materialien. Denkbar ist es aber auch, die Sterilverpackung aus nur einem Material auszubilden, insbesondere auch einstückig. Insbesondere kann das Metall ein oder mehrere Stähle umfassen, beispielsweise Edelstähle mit den Werkstoffnummern 1.4021, 1.4024, 1.4028, 1.4031, 1.4034, 1.4104, 1.4112, 1.4116, 1.4121, 1.4125, 1.4301, 1.4305, 1.4310, 1.4401, 1.4435, 1.4441, 1.4541, 1.4543, 1.4568 und/oder 1.4571. Der Kunststoff kann insbesondere Polyetheretherketon (PEEK) sein oder enthalten.

Um das Beschicken der Sterilverpackung mit mindestens einem Implantat so einfach wie möglich zu gestalten, ist es günstig, wenn die Sterilverpackung einen Behälter zum Aufnehmen mindestens eines Implantats mit einer Einführöffnung und ein Verschlusselement zum Verschließen der Einführöffnung umfasst. Das mindestens eine Implantat durch die Einführöffnung in den Behälter eingeführt werden, welcher wiederum mittels des Verschlusselements verschlossen werden kann. Das Beschicken der Sterilverpackung mit dem mindestens einen Implantat erfolgt vorzugsweise in einer sterilen Umgebung. Optional ist es auch möglich, das mindestens eine Implantat in der die Lagerstellung einnehmenden Sterilverpackung nochmals zu sterilisieren, beispielsweise durch Bestrahlen mit Gamma-Strahlung.

Günstigerweise sind der Behälter und das Verschlusselement in der Lagerstellung kraft- und/oder formschlüssig miteinander verbunden. Dadurch kann sichergestellt werden, dass sich das Verschlusselement nicht in unerwünschter Weise vom Behälter lösen kann, was zur Folge habe könnte, dass das in der Sterilverpackung verpackte, mindestens eine Implantat verunreinigt oder unsteril wird.

Auf besonders einfache Weise lassen sich der Behälter und das Verschlusselement kraftschlüssig miteinander verbinden, wenn sie in der Lagerstellung miteinander verklebt und/oder verschweißt sind. Insbesondere ist es vorteilhaft, den Behälter und das Verschlusselement mittels eines Lasers zu verschweißen. Bei einer Verklebung kommen vorzugsweise Klebstoffe zum Einsatz, die druck- und hitzebeständig sowie lösungsmittelbeständig sind, um übliche Reinigungsprozesse zur Wiederaufbereitung der Sterilverpackung durchführen zu können.

Zum sicheren Verbinden des Behälters und des Verschlusselements in der Lagerstellung ist vorzugsweise mindestens eine Schweißnaht vorgesehen. Günstigerweise ist die mindestens eine Schweißnaht in sich geschlossen und ringförmig ausgebildet. Damit kann beispielsweise eine zusätzliche Sterilbarriere geschaffen werden. Werden zwei in sich geschlossene Schweißnähte vorgesehen, kann also zum Beispiel eine redundante, das heißt doppelte, Sterilbarriere ausgebildet werden.

Das Öffnen der Sterilverpackung wird besonders einfach, wenn diese mindestens eine Sollbruchstelle zum Öffnen aufweist. Dies gestattet beispielsweise das Entnehmen des mindestens einen Implantats aus der Sterilverpackung durch gezieltes Zerstören der Sollbruchstelle.

Günstigerweise ist die Sollbruchstelle am Behälter und/oder am Verschlusselement ausgebildet. Dies gestattet es, wahlweise einen Teil des Verschlusselements oder einen Teil des Behälters zu entfernen, um so eine Entnahmeöffnung zu schaffen.

Besonders einfach ausbilden lässt sich die Sollbruchstelle durch eine Schwächung des den Behälter und/oder das Verschlusselement ausbildenden Materials. Beispielsweise kann eine Wandstärke des Behälters und/oder des Verschlusselements zur Ausbildung der Sollbruchstelle etwas verringert sein. Günstigerweise ermöglicht es die Sollbruchstelle, die Sterilverpackung ohne Zuhilfenahme von Werkzeugen zu öffnen.

Günstigerweise weist eine aus einem Kunststoff hergestellte Sterilverpackung eine Mindestwandstärke von etwa 2 mm auf. Vorzugsweise beträgt eine maximale Wandstärke der aus einem Kunststoff hergestellten Sterilverpackung etwa 7 mm. Günstigerweise weist eine aus einem Metall, insbesondere einem Stahl, hergestellte Sterilverpackung eine Mindestwandstärke von etwa 0,5 mm auf. Vorzugsweise beträgt eine maximale Wandstärke der aus einem Metall hergestellten Sterilverpackung etwa 5 mm.

Die Herstellung der Sterilverpackung vereinfacht sich weiter, wenn die Soll-bruchstelle eine Nut umfasst. Diese kann insbesondere umlaufend ausgebildet sein, beispielsweise auf einer äußeren Oberfläche des Behälters oder des Verschlusselements. Günstigerweise ist die Nut von der Sterilverpackung weg weisend geöffnet. Eine solche Nut lässt sich besonders einfach an der Sterilverpackung ausbilden, beispielsweise durch Fräsen oder entsprechende Formgebung im Rahmen eines Gießprozesses zur Herstellung des Verschlusselements oder des Behälters.

Um eine möglichst definierte Eröffnung der Sterilverpackung zu gestatten, ist es vorteilhaft, wenn die Nut einen keilförmigen Querschnitt aufweist.

Um das Verschließen des Behälters auf einfache Weise zu ermöglichen, sind der Behälter und das Verschlusselement vorzugsweise miteinander verschraubbar ausgebildet.

Das Verschrauben des Behälters und des Verschlusselements miteinander kann auf besonders einfache Weise beispielsweise dadurch realisiert werden, dass der Behälter ein Behälterinnengewinde oder ein Behälteraußengewinde aufweist und dass das Verschlusselement ein zum Behälterinnengewinde korrespondierendes Verschlusselementaußengewinde oder ein zum Behälteraußengewinde korrespondierendes Verschlusselementinnengewinde aufweist.

Die Stabilität der Verpackung sowie deren Keimdichtigkeit kann insbesondere dadurch verbessert werden, dass das Verschlusselement einen in der Lagerstellung in die Einführöffnung eintauchenden Vorsprung aufweist. Der Vorsprung kann insbesondere in Form eines Stopfens ausgebildet sein. Günstigerweise ist das Verschlusselement in diesem Fall aus einem Material gebildet, welches eine gewisse Elastizität aufweist, um eine Abdichtung der Einführöffnung zu gewährleisten.

Günstig kann es ferner sein, wenn das Verschlusselement einen auf einem Rand oder einer Randfläche der Einführöffnung in der Lagerstellung aufliegenden Flansch aufweist. So kann auf einfache Weise eine Abdichtung zwischen dem Verschlusselement und dem Rand des Behälters erreicht werden.

Um die Einführöffnung beziehungsweise einen Rand derselben zu schützen, ist es vorteilhaft, wenn das Verschlusselement einen den Behälter im Bereich der Einführöffnung ringförmig umgebenden Randabschnitt aufweist. Das Verschlusselement kann also insbesondere in Form einer Kappe ausgebildet sein, die die Einführöffnung übergreift. Vorzugsweise sind sowohl die Einführöffnung als auch das Verschlusselement rotationssymmetrisch oder im Wesentlichen rotationssymmetrisch ausgebildet.

Günstigerweise ist der Wandabschnitt zylindrisch oder im Wesentlichen zylindrisch ausgebildet. So kann er beispielsweise korrespondierend zu einem zylindrisch ausgebildeten Wandabschnitt der Einführöffnung bemessen sein, um die Einführöffnung sicher zu verschließen.

Kostengünstig herstellbar sowie einfach und sicher vom Behälter entfernbar ist das Verschlusselement beispielsweise dadurch, dass es in Form eines Kronkorkens ausgebildet ist.

Gemäß einer bevorzugten Ausführungsform der Erfindung kann ein Dichtelement zum Abdichten des Behälters und des Verschlusselements relativ zueinander vorgesehen sein. Beispielsweise kann das Dichtelement bei einem Kronkorken in Form einer auf einer Innenseite desselben angeordneten Einlage ausgebildet sein, welche eine gewisse Elastizität aufweist. Das Dichtelement kann insbesondere aus sterilisierbaren Kunststoffen wie beispielsweise Polyvinylchlorid (PVC), Polyethylen (PE) oder Polyetheretherketon (PEEK) ausgebildet sein.

Günstigerweise liegt das Dichtelement in der Lagerstellung an einer Behälterdichtfläche des Behälters und an einer Verschlusselementdichtfläche des Verschlusselements an. Beispielsweise kann die Behälterdichtfläche durch einen vom Behälter weg weisenden, die Einführöffnung umgebenden Rand gebildet werden, die Verschlusselementdichtfläche durch einen korrespondierenden Flansch des Verschlusselements.

Um das Verschließen der Sterilverpackung zu erleichtern, ist es vorteilhaft, wenn das Dichtelement am Behälter oder am Verschlusselement gehalten oder befestigt ist. Beispielsweise kann es kraft- und/oder formschlüssig am Behälter oder am Verschlusselement gehalten sein. Insbesondere kann das Dichtelement am Behälter oder am Verschlusselement angeklebt oder angeschweißt sein.

Besonders einfach und kostengünstig hergestellt werden kann die Sterilverpackung, wenn das Verschlusselement aus einer Folie oder einem dünnen Blech hergestellt ist. Insbesondere kann die Folie eine Metall- oder eine Kunststofffolie oder eine mehrlagige Folie aus mindestens jeweils einer Metall- und/ oder Kunststoffschicht sein. Beispielsweise lässt sich eine Folie über eine Einführöffnung des Behälters spannen und durch Punkt- oder Laserschweißen mit dem Behälter verbinden. Alternativ können die Folie oder das dünne Blech auch mit dem Behälter verklebt werden.

Besonders einfach herstellbar ist der Behälter, wenn er eine zylindrische oder im Wesentlichen zylindrische Wand aufweist. Der Behälter kann optional auch insgesamt rotationssymmetrisch ausgebildet sein. Beispielsweise kann er aus einem rohrförmigen Rohling gebildet werden durch einseitiges Zuhämmern desselben und Ausbilden eines entsprechenden Verschlusselements zum Verschließen des anderen Rohrendes.

Gemäß einer weiteren bevorzugten Ausführungsform der Erfindung kann mindestens ein Werkzeugelement zum mindestens teilweisen Entfernen des Verschlusselements zum Öffnen der Sterilverpackung vorgesehen sein. Das mindestens eine Werkzeugelement kann insbesondere einstückig mit dem Verschlusselement ausgebildet oder mit diesem dauerhaft verbunden sein. Selbstverständlich kann optional das mindestens eine Werkzeugelement auch derart angeordnet und ausgebildet sein, dass ein Teil des Behälters entfernbar ist zum Eröffnen der Sterilverpackung.

Eine besonders einfache und sichere Handhabung eines Implantats ermöglicht eine Sterilverpackung, wenn das mindestens eine Werkzeugelement in Form eines Zugglieds ausgebildet ist. Beispielsweise lässt sich das Zugglied von einem Anwender fassen und so eine entsprechende Zugkraft auf einen Teil der Sterilverpackung ausüben, um beispielsweise die Sterilverpackung durch Ausbilden einer Entnahmeöffnung zu eröffnen. Die Entnahmeöffnung kann vordefiniert werden, beispielsweise durch eine Sollbruchstelle, welche insbesondere in Form oben beschriebener, vorteilhafter Ausführungsformen von Sollbruchstellen ausgebildet sein kann. Besonders einfach handzuhaben ist ein Zugglied in Form einer Zuglasche. Beispielsweise kann so ein Finger durch die Lasche gesteckt und die Sterilverpackung sicher eröffnet werden.

Um sicher zu stellen, dass nicht versehentlich unsterile Sterilverpackungen und damit auch unsterile Implantate in den Sterilbereich während eines chirurgischen Eingriffs gelangen können, ist es vorteilhaft, wenn die Sterilverpackung eine Anzeigeeinrichtung umfasst zum Anzeigen, ob die Sterilverpackung geöffnet oder beschädigt wurde. Die Anzeigeeinrichtung kann insbesondere mechanischer Natur sein und beispielsweise eine Sollbruchstelle umfassen, die von einem Anwender vor Verwendung der Sterilverpackung und des darin enthaltenden Implantats geprüft werden kann, zum Beispiel durch eine Sichtprüfung.

Günstig ist es, wenn die Anzeigeeinrichtung einen Sauerstoffindikator umfasst. Vorzugsweise ist der Sauerstoffindikator im Aufnahmeraum angeordnet. Er kann insbesondere derart ausgebildet sein, dass er bei in Kontakt Treten mit Sauerstoff seine Farbe ändert, um so einem Anwender zu signalisieren, dass die Sterilverpackung undicht und folglich nicht mehr steril ist.

Ferner kann es vorteilhaft sein, wenn die Sterilverpackung mindestens ein Speicherelement zum Speichern von Informationen zur Charakterisierung mindestens eines in der Sterilverpackung verpackten Implantats umfasst. Beispielsweise lassen sich auf dem Speicherelement Daten betreffend die Art des Implantats, dessen Größe, dessen Herstelldatum oder auch dessen Chargenbezeichnung speichern. Insbesondere kann das Speicherelement zur Dokumentation eines chirurgischen Eingriffs verwendet werden, um später nachvollziehen zu können, welches oder welche Implantate tatsächlich implantiert wurden.

Die Handhabung der Sterilverpackung sowie der Ablauf eines chirurgischen Eingriffs können insbesondere dadurch optimiert werden, dass das Speicherelement berührungslos auslesbar ist. Beispielsweise kann es optisch oder per Funk auslesbar ausgebildet sein.

Besonders einfach und kostengünstig lässt sich ein Speicherelement vorsehen, wenn es in Form eines Barcodes oder einer Farbkodierung ausgebildet oder Teil eines RFID-Chips ist. Derartige Speicherelemente lassen sich optisch, beispielsweise mit einem Barcode-Scanner, oder mittels eines RFID-Chip-Auslesegeräts auslesen. Die Ausbildung in Form einer Farbkodierung hat den Vorteil, dass es einem Anwender direkt möglich ist, den Inhalt der Sterilverpackung anhand der Farbkodierung zu erkennen.

Das Speicherelement lässt sich besonders einfach und kostengünstig herstellen, wenn das Verschlusselement farbig ausgebildet ist zur Ausbildung der Farbkodierung. Dadurch lassen sich beispielsweise unterschiedliche Implantatgrößen anhand der Farbe auswählen.

Vorteilhaft ist es, wenn die medizinische Sterilverpackung mindestens ein im Aufnahmeraum in der Lagerstellung verpacktes Implantat umfasst. Auf diese Weise ist es einem Hersteller von Implantaten möglich, ein Implantat bereits steril in der Sterilverpackung zu verpacken und somit steril auszuliefern. Die Sterilverpackung mit dem Implantat muss jedoch nicht zwingend nochmals steril verpackt werden. Es ist grundsätzlich möglich, das in der Sterilverpackung verpackte Implantat vor der eigentlichen Nutzung vor Ort, beispielsweise in einem Krankenhaus, zu sterilisieren. Dies vermindert den Verpackungs- und Lagerungsaufwand.

Ferner betrifft die vorliegende Erfindung die Verwendung einer medizinischen Sterilverpackung in Form einer der oben beschriebenen, bevorzugten Ausführungsformen, zum sterilen Verpacken mindestens eines Implantats, welches ausgebildet ist zum temporären oder dauerhaften Einsetzen in einen menschlichen oder tierischen Körper.

Eine medizinische Sterilverpackung zu diesem Zweck zu verwenden, hat den Vorteil, dass eine aufwendige Aufbereitung des Implantats selbst entfallen kann, denn es muss lediglich die das Implantat umgebende Sterilverpackung aufbereitet werden, um ein während eines chirurgischen Eingriffs nicht benötigtes Implantat für einen späteren Eingriff wieder aufbereiten zu können.

Die vorstehende Beschreibung umfasst somit insbesondere die nachfolgend explizit aufgeführten Ausführungsformen einer medizinischen Sterilverpackung sowie einer Verwendung einer medizinischen Sterilverpackung:
1. Medizinische Sterilverpackung (10), welche einen Aufnahmeraum (28) für mindestens ein Implantat (26; 26a, 26b), welches ausgebildet ist zum temporären oder dauerhaften Einsetzen in einen menschlichen oder tierischen Körper, definiert, welcher Aufnahmeraum (28) in einer Lagerstellung der Sterilverpackung (10) steril und keimdicht verschlossen ist zum sterilen Verpacken des mindestens einen Implantats (26; 26a, 26b), dadurch gekennzeichnet, dass die Sterilverpackung (10) aus einem oder mehreren reinig- und sterilisierbaren Materialien hergestellt ist.
2. Medizinische Sterilverpackung nach Satz 1, dadurch gekennzeichnet, dass das oder die Materialien lösungsmittelbeständig sind gegen Reinigungs- und Lösungsmittel mit einem pH-Wert in einem Bereich von etwa 2 bis etwa 12, insbesondere in einem Bereich von etwa 3 bis etwa 11.
3. Medizinische Sterilverpackung nach Satz 1 oder 2, dadurch gekennzeichnet, dass das oder die Materialien wärme-/hitzebeständig sind bis zu einer Temperatur von etwa 150°C, insbesondere bis zu einer Temperatur von etwa 130°C.
4. Medizinische Sterilverpackung nach einem der voranstehenden Sätze, dadurch gekennzeichnet, dass das oder die Materialien druckbeständig sind bei Drücken bis mindestens etwa 5 bar, insbesondere bei Drücken bis mindestens etwa 3 bar.
5. Medizinische Sterilverpackung nach einem der voranstehenden Sätze, dadurch gekennzeichnet, dass die Sterilverpackung (10) formstabil ist, insbesondere bei Temperaturen und Drücken in Bereichen gemäß der Sätze 3 oder 4.
6. Medizinische Sterilverpackung nach einem der voranstehenden Sätze, dadurch gekennzeichnet, dass das oder die Materialien transparent oder transluzent sind.
7. Medizinische Sterilverpackung nach einem der voranstehenden Sätze, dadurch gekennzeichnet, dass das oder die Materialien Glas, Metall, Kunststoff oder Keramik sind.
8. Medizinische Sterilverpackung nach einem der voranstehenden Sätze, dadurch gekennzeichnet, dass die Sterilverpackung (10) einen Behälter (12) zum Aufnehmen mindestens eines Implantats (26; 26a, 26b) mit einer Einführöffnung (24) und ein Verschlusselement (14) zum Verschließen der Einführöffnung (24) umfasst.
9. Medizinische Sterilverpackung nach Satz 8, dadurch gekennzeichnet, dass der Behälter (12) und das Verschlusselement (14) in der Lagerstellung kraft- und/oder formschlüssig miteinander verbunden sind.
10. Medizinische Sterilverpackung nach Satz 8 oder 9, dadurch gekennzeichnet, dass der Behälter (12) und das Verschlusselement (14) in der Lagerstellung miteinander verklebt und/oder verschweißt sind, insbesondere mit einem Laser verschweißt.
11. Medizinische Sterilverpackung nach einem der Sätze 8 bis 10, dadurch gekennzeichnet, dass mindestens eine Schweißnaht (60; 60a, 60b) vorgesehen ist zum Verbinden des Behälters (12) und des Verschlusselements (14) in der Lagerstellung, insbesondere mindestens eine in sich geschlossene, ringförmige Schweißnaht (60; 60a, 60b).
12. Medizinische Sterilverpackung nach einem der voranstehenden Sätze, dadurch gekennzeichnet, dass die Sterilverpackung (10) mindestens eine Sollbruchstelle (66) zum Öffnen aufweist.
13. Medizinische Sterilverpackung nach Satz 12, dadurch gekennzeichnet, dass die Sollbruchstelle (66) am Behälter (12) und/oder am Verschlusselement (14) ausgebildet ist.
14. Medizinische Sterilverpackung nach Satz 12 oder 13, dadurch gekennzeichnet, dass die Sollbruchstelle (66) durch eine Schwächung des den Behälter (12) und/oder das Verschlusselement (14) ausbildenden Materials ausgebildet ist.
15. Medizinische Sterilverpackung nach einem der Sätze 12 bis 14, dadurch gekennzeichnet, dass die Sollbruchstelle (66) eine Nut (70) umfasst, insbesondere eine von der Sterilverpackung (10) weg weisend geöffnete Nut.
16. Medizinische Sterilverpackung nach Satz 15, dadurch gekennzeichnet, dass die Nut (70) einen keilförmigen Querschnitt aufweist.
17. Medizinische Sterilverpackung nach einem der Sätze 8 bis 16, dadurch gekennzeichnet, dass der Behälter (12) und das Verschlusselement (14) miteinander verschraubbar ausgebildet sind.
18. Medizinische Sterilverpackung nach Satz 17, dadurch gekennzeichnet, dass der Behälter (12) ein Behälterinnengewinde oder ein Behälteraußengewinde (44) aufweist und dass das Verschlusselement (14) ein zum Behälterinnengewinde korrespondierendes Verschlusselementaußengewinde oder ein zum Behälteraußengewinde (44) korrespondierendes Verschlusselementinnengewinde (46) aufweist.
19. Medizinische Sterilverpackung nach einem der Sätze 8 bis 18, dadurch gekennzeichnet, dass das Verschlusselement (14) einen in der Lagerstellung in die Einführöffnung (24) eintauchenden Vorsprung (34) aufweist.
20. Medizinische Sterilverpackung nach einem der Sätze 8 bis 19, dadurch gekennzeichnet, dass das Verschlusselement (14) einen auf einem Rand (22) der Einführöffnung (24) in der Lagerstellung aufliegenden Flansch aufweist.
21. Medizinische Sterilverpackung nach einem der Sätze 8 bis 20, dadurch gekennzeichnet, dass das Verschlusselement (14) einen den Behälter (12) im Bereich der Einführöffnung (24) ringförmig umgebenden Wandabschnitt (48) aufweist.
22. Medizinische Sterilverpackung nach Satz 21, dadurch gekennzeichnet, dass der Wandabschnitt (48) zylindrisch oder im Wesentlichen zylindrisch ausgebildet ist.
23. Medizinische Sterilverpackung nach einem der Sätze 8 bis 22, dadurch gekennzeichnet, dass das Verschlusselement (14) in Form eines Kronkorkens (112) ausgebildet ist.
24. Medizinische Sterilverpackung nach einem der Sätze 8 bis 23, gekennzeichnet durch ein Dichtelement (106) zum Abdichten des Behälters (12) und des Verschlusselements (14) relativ zueinander.
25. Medizinische Sterilverpackung nach Satz 24, dadurch gekennzeichnet, dass das Dichtelement (106) in der Lagerstellung an einer Behälterdichtfläche (40) des Behälters (12) und an einer Verschlusselementdichtfläche (38) des Verschlusselements (14) anliegt.
26. Medizinische Sterilverpackung nach Satz 24 oder 25, dadurch gekennzeichnet, dass das Dichtelement (106) am Behälter (12) oder am Verschlusselement (14) gehalten oder befestigt ist.
27. Medizinische Sterilverpackung nach einem der Sätze 8 bis 26, dadurch gekennzeichnet, dass das Verschlusselement (14) aus einer Folie hergestellt ist, insbesondere einer Metall- und/oder Kunststofffolie.
28. Medizinische Sterilverpackung nach einem der Sätze 8 bis 27, dadurch gekennzeichnet, dass der Behälter (12) eine zylindrische oder im Wesentlichen zylindrische Wand (68) aufweist.
29. Medizinische Sterilverpackung nach einem der Sätze 8 bis 28, dadurch gekennzeichnet, dass mindestens ein Werkzeugelement (84) vorgesehen ist zum mindestens teilweisen Entfernen des Verschlusselements (14) zum Eröffnen der Sterilverpackung (10).
30. Medizinische Sterilverpackung nach Satz 29, dadurch gekennzeichnet, dass das mindestens eine Werkzeugelement (84) in Form eines Zugglieds (86) ausgebildet ist, insbesondere in Form einer Zuglasche (88).
31. Medizinische Sterilverpackung nach einem der voranstehenden Sätze, gekennzeichnet durch eine Anzeigeeinrichtung (102) zum Anzeigen, ob die Sterilverpackung (10) geöffnet oder beschädigt wurde.
32. Medizinische Sterilverpackung nach Satz 31, dadurch gekennzeichnet, dass die Anzeigeeinrichtung (102) einen Sauerstoffindikator (104) umfasst.
33. Medizinische Sterilverpackung nach einem der voranstehenden Sätze, gekennzeichnet durch mindestens ein Speicherelement (98) zum Speichern von Informationen zur Charakterisierung mindestens eines in der Sterilverpackung (10) verpackten Implantats (26; 26a, 26b).
34. Medizinische Sterilverpackung nach Satz 33, dadurch gekennzeichnet, dass Speicherelement (98) berührungslos auslesbar ist.
35. Medizinische Sterilverpackung nach Satz 33 oder 34, dadurch gekennzeichnet, dass das Speicherelement (98) in Form eines Barcodes oder einer Farbkodierung ausgebildet oder Teil eines RFID-Chips (100) ist.
36. Medizinische Sterilverpackung nach Satz 35, dadurch gekennzeichnet, dass das Verschlusselement (14) farbig ausgebildet ist zur Ausbildung der Farbkodierung.
37. Medizinische Sterilverpackung nach einem der voranstehenden Sätze, gekennzeichnet durch mindestens ein im Aufnahmeraum (28) in der Lagerstellung verpacktes Implantat (26; 26a, 26b).
38. Verwendung einer medizinischen Sterilverpackung (10) nach einem der Sätze 1 bis 36 zum sterilen Verpacken mindestens eines Implantats (26; 26a, 26b), welches ausgebildet ist zum temporären oder dauerhaften Einsetzen in einen menschlichen oder tierischen Körper.

Die nachfolgende Beschreibung bevorzugter Ausführungsformen der Erfindung dient im Zusammenhang mit der Zeichnung der näheren Erläuterung. Es zeigen:
- Figur 1a:: eine schematische Schnittansicht eines ersten Ausführungsbeispiels einer Sterilverpackung vor dem Einbringen eines Implantats;
- Figur 1b:: eine schematische Schnittansicht des ersten Ausführungsbeispiels einer Sterilverpackung aus Figur 1a nach dem Einbringen des Implantats und Verschließen;
- Figur 2a:: eine schematische Schnittansicht eines zweiten Ausführungsbeispiels einer Sterilverpackung vor dem Einbringen eines Implantats;
- Figur 2b:: eine schematische Schnittansicht des zweiten Ausführungsbeispiels einer Sterilverpackung aus Figur 2a nach dem Einbringen des Implantats und Verschließen;
- Figur 3a:: eine schematische Schnittansicht eines dritten Ausführungsbeispiels einer Sterilverpackung vor dem Einbringen eines Implantats;
- Figur 3b:: eine schematische Schnittansicht des dritten Ausführungsbeispiels einer Sterilverpackung aus Figur 3a nach dem Einbringen des Implantats und Verschließen;
- Figur 4a:: eine schematische Schnittansicht eines vierten Ausführungsbeispiels einer Sterilverpackung vor dem Einbringen eines Implantats;
- Figur 4b:: eine schematische Schnittansicht des vierten Ausführungsbeispiels einer Sterilverpackung aus Figur 4a nach dem Einbringen des Implantats und Verschließen;
- Figur 5a:: eine schematische Ansicht eines fünften Ausführungsbeispiels einer verschlossenen Sterilverpackung mit Implantat;
- Figur 5b:: eine schematische Ansicht des fünften Ausführungsbeispiels einer Sterilverpackung aus Figur 5a nach dem Öffnen und Entnehmen des Implantats;
- Figur 6a:: eine schematische Ansicht eines sechsten Ausführungsbeispiels einer verschlossenen Sterilverpackung mit Implantat;
- Figur 6b:: eine schematische Ansicht des sechsten Ausführungsbeispiels einer Sterilverpackung aus Figur 6a nach dem Öffnen und Entnehmen des Implantats;
- Figur 7a:: eine schematische Schnittansicht eines siebten Ausführungsbeispiels einer Sterilverpackung vor dem Einbringen eines Implantats;
- Figur 7b:: eine schematische Schnittansicht des siebten Ausführungsbeispiels einer Sterilverpackung aus Figur 7a nach dem Einbringen des Implantats und Verschließen;
- Figur 8a:: eine schematische Schnittansicht eines achten Ausführungsbeispiels einer Sterilverpackung vor dem Einbringen eines Implantats;
- Figur 8b:: eine schematische Schnittansicht des achten Ausführungsbeispiels einer Sterilverpackung aus Figur 8a nach dem Einbringen des Implantats und Verschließen;
- Figur 9:: eine schematische Schnittansicht eines neunten Ausführungsbeispiels einer Sterilverpackung nach dem Einbringen des Implantats und Verschließen;
- Figur 10:: eine schematische Schnittansicht eines zehnten Ausführungsbeispiels einer Sterilverpackung nach dem Einbringen des Implantats und Verschließen;
- Figur 11:: eine schematische Schnittansicht eines elften Ausführungsbeispiels einer Sterilverpackung nach dem Einbringen von zwei Implantaten und Verschließen;
- Figur 12a:: eine schematische Schnittansicht eines zwölften Ausführungsbeispiels einer Sterilverpackung aus Figur 8a nach dem Einbringen des Implantats und Verschließen; und
- Figur 12b:: eine schematische Ansicht der Entnahmeöffnung des zwölften Ausführungsbeispiels einer Sterilverpackung aus Figur 12a nach dem Öffnen und Entnehmen des Implantats.

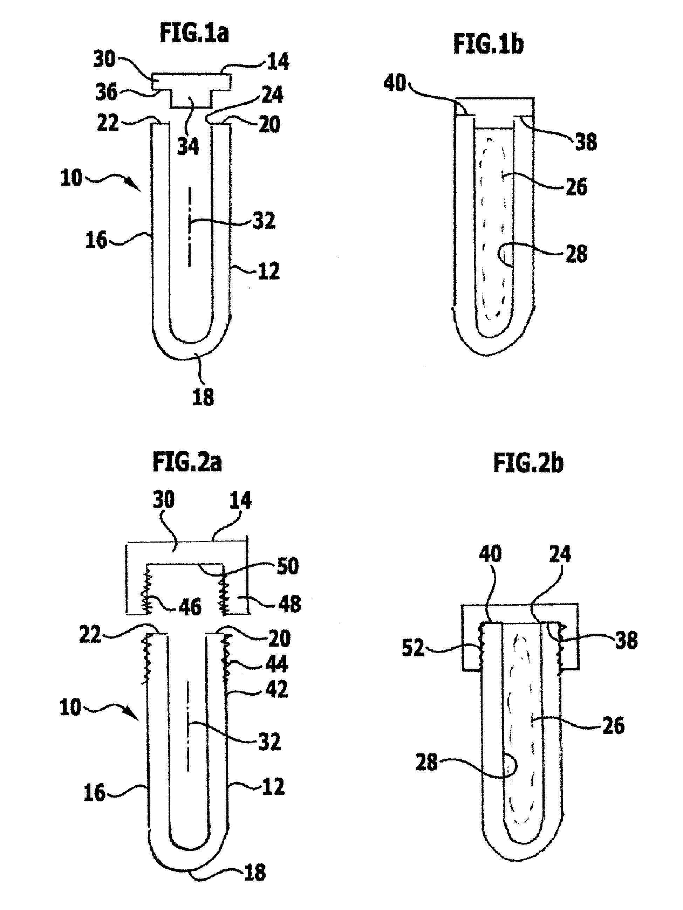

In Figur 1 ist schematisch ein erstes Ausführungsbeispiel einer insgesamt mit dem Bezugszeichen 10 bezeichneten Sterilverpackung dargestellt. Es umfasst einen Behälter 12 und ein Verschlusselement 14. Sowohl der Behälter 12 als auch das Verschlusselement 14 sind vorzugsweise rotationssymmetrisch ausgebildet. Der Behälter 12 ist aus einem Rohr 16 hergestellt, welches an seinem einen Ende 18 durch Zuhämmern verschlossen ist, sodass eine im Wesentlichen halbe Hohlkugel ausgebildet ist. Das gegenüberliegende Ende 20 definiert eine vom Ende 18 weg weisende, ringförmig geschlossene Randfläche 22, welche eine Einführöffnung 24 umgibt, durch welche ein Implantat 26, welches ausgebildet ist zum temporären oder dauerhaften Einsetzen in einen menschlichen oder tierischen Körper, in einen von der Sterilverpackung 10 definierten Aufnahmeraum 28 eingeführt werden kann.

Das Verschlusselement 14 ist in Form einer kreisförmigen Scheibe 30 ausgebildet, von welcher sich ein koaxial zu einer vom Behälter 12 definierten Längsachse 32 abstehender, zylindrischer Vorsprung 34 weg erstreckt. Der Vorsprung 34 ist so bemessen, dass er in die Einführöffnung 24 formschlüssig einführbar ist. Der Vorsprung 34 grenzt an eine Ringfläche 36 der Scheibe 30 an, welche eine Verschlusselementdichtfläche 38 definiert, die in einer Lagerstellung der Sterilverpackung, in welcher sie steril und keimdicht verschlossen ist zum sterilen Verpacken des Implantats 26, an der eine Behälterdichtfläche 40 ausbildenden Randfläche 22 anliegt.

Der Behälter 12 und das Verschlusselement 14 können optional miteinander kraftschlüssig und/oder stoffschlüssig verbunden sein, beispielsweise durch Kleben oder Schweißen. Der Behälter 12 und das Verschlusselement 14 können aus demselben Material hergestellt sein oder aus unterschiedlichen Materialien. Der Behälter 12 kann beispielsweise aus einem Edelstahl gebildet sein, das Verschlusselement 14 ebenfalls aus einem Edelstahl oder aus einem Kunststoff.

In Figur 1b ist das erste Ausführungsbeispiel der Sterilverpackung 10 schematisch in der Lagerstellung dargestellt.

Ein zweites Ausführungsbeispiel einer Sterilverpackung 10 ist schematisch in Figur 2a dargestellt. Der Aufbau des Behälters 12 entspricht im Wesentlichen dem Aufbau des Behälters 12 des in den Figuren 1a und 1b schematisch dargestellten ersten Ausführungsbeispiels der Sterilverpackung 10. Der Übersichtlichkeit halber sind identische Elemente und Teile des bereits beschriebenen Ausführungsbeispiels sowie der nachfolgend beschriebenen Ausführungsbeispiele von Sterilverpackungen 10 mit identischen Bezugszeichen versehen.

Auf einer Außenseite 42 weist der Behälter 12 einen kurzen Außengewindeabschnitt 44 auf. Dieser ist korrespondierend zu einem Innengewindeabschnitt 46 an einem zylindrischen Abschnitt 48 des Verschlusselements 14 ausgebildet. Der Abschnitt 48 ist vom Behälter 12 weg weisend mit einer kreisförmigen Scheibe 30 verschlossen. Eine in Richtung auf den Behälter 12 weisende Innenfläche 50 des Verschlusselements 14 bildet die Verschlusselementdichtfläche 38, welche an der Behälterdichtfläche 40 anliegt.

Das Implantat 26 kann durch die Einführöffnung 24 bei abgenommenem Verschlusselement 14 eingeführt werden. Anschließend kann das Verschlusselement 14 auf den Behälter 12 aufgeschraubt werden. Zum Sichern der Schraubverbindung sowie zum optionalen Abdichten kann auf den Außengewindeabschnitt 44 und/oder den Innengewindeabschnitt 46 etwas Klebstoff 52 aufgebracht werden, um das Verschlusselement 14 kraft- beziehungsweise stoffschlüssig mit dem Behälter 12 zu verbinden.

Der Behälter 12 und das Verschlusselement 14 können beispielsweise beide aus einem Edelstahl hergestellt sein. Alternativ ist es auch möglich, das Verschlusselement 14 aus Glas oder einem Kunststoff herzustellen, welcher beispielsweise abhängig von der Art des in der Sterilverpackung 10 zu verpackenden Implantats 26 mit einer bestimmten Farbe eingefärbt sein kann, um so eine Farbkodierung für das Implantat 26 zu bilden.

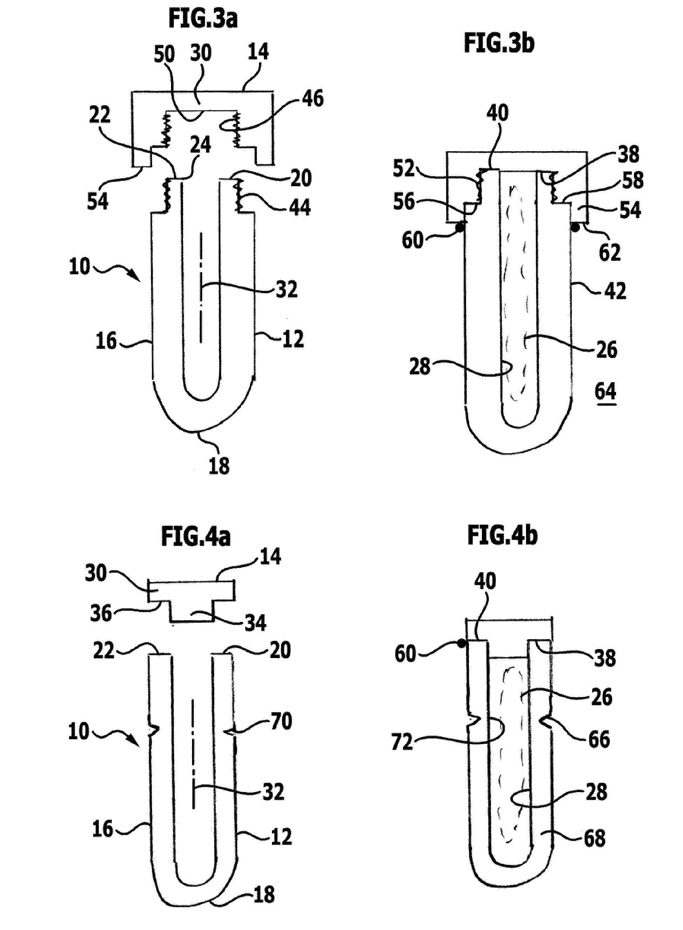

In den Figuren 3a und 3b ist schematisch ein drittes Ausführungsbeispiel einer Sterilverpackung 10 dargestellt. Es entspricht in seinem grundsätzlichen Aufbau dem in den Figuren 2a und 2b schematisch dargestellten zweiten Ausführungsbeispiel der Sterilverpackung 10. Der Unterschied zum zweiten Ausführungsbeispiel besteht darin, dass sich ein Außendurchmesser des Behälters 12 einstufig zum Ende 20 hin verringert, und zwar auf einer Länge, die einer Länge des Außengewindeabschnitts 44 entspricht. Am Verschlusselement 14 erstreckt sich vom Abschnitt 48 ein konzentrisch zur Längsachse 32 ausgebildeter, zweiter zylindrischer Abschnitt 54 weg in Richtung auf den Behälter 12 hin weisend, welcher einen Innendurchmesser aufweist, der einem Außendurchmesser des Behälters 12 entspricht, wo dieser gewindefrei ist. In der Lagerstellung, in welcher das Verschlusselement 14 auf den Behälter 12 aufgeschraubt ist, übergreift der Abschnitt 54 die gewindefreie Außenseite 42. Am Verschlusselement 14 ist eine in Richtung auf den Behälter 12 weisende Ringfläche 46 ausgebildet, welche an einer in Richtung auf das Verschlusselement 14 hin weisenden Ringfläche 48 des Behälters 12 in der Lagerstellung anliegt. Der Außengewindeabschnitt 44 erstreckt sich zwischen der Ringfläche 58 und der Randfläche 22.

Nach dem Einbringen des Implantats 26 in den Aufnahmeraum 28 wird das Verschlusselement 14 auf den Behälter 12 aufgeschraubt. Zum Fixieren des Verschlusselements 14 in der in Figur 3b dargestellten Lagerstellung kann optional eine Schweißnaht im Übergangsbereich zwischen einer in Richtung auf das Ende 18 hin weisenden Ringfläche 62 des Abschnitts 54 und der Außenseite 42 angebracht werden, beispielsweise durch Laserschweißen. Die Schweißnaht 60 kann insbesondere in sich geschlossen ringförmig ausgebildet werden und so zusätzlich den Aufnahmeraum 28 gegenüber einer Umgebung 64 der Sterilverpackung 10 abdichten. Alternativ oder zusätzlich kann auch ein Klebstoff 52 eingesetzt werden, um den Außengewindeabschnitt 44 mit dem Innengewindeabschnitt 46 zu verkleben.

In den Figuren 4a und 4b ist schematisch ein viertes Ausführungsbeispiel einer Sterilverpackung 10 dargestellt. Es entspricht in seinem Aufbau im Wesentlichen dem in den Figuren 1a und 1b dargestellten Ausführungsbeispiel der Sterilverpackung 10 und unterscheidet sich von diesem lediglich dadurch, dass am Behälter 12 eine Sollbruchstelle 66 vorgesehen ist. Die Sollbruchstelle 66 wird durch eine Schwächung des den Behälter 12 ausbildenden Materials gebildet, und zwar durch eine Verringerung einer Dicke einer Wand 68 des Behälters 12. Die Verringerung der Dicke der Wand 68 zur Ausbildung der Sollbruchstelle 66 wird realisiert durch eine den Behälter 12 auf seiner Außenseite 42 ringförmig umgebende Nut 70, welche einen keilförmigen Querschnitt aufweist.

Zum Öffnen der Sterilverpackung 10 kann die Sollbruchstelle 66 durch Abdrehen oder Abknicken durchtrennt und dann das Implantat 26 durch die im Bereich der Sollbruchstelle 66 ausgebildete Entnahmeöffnung 72 entnommen werden.

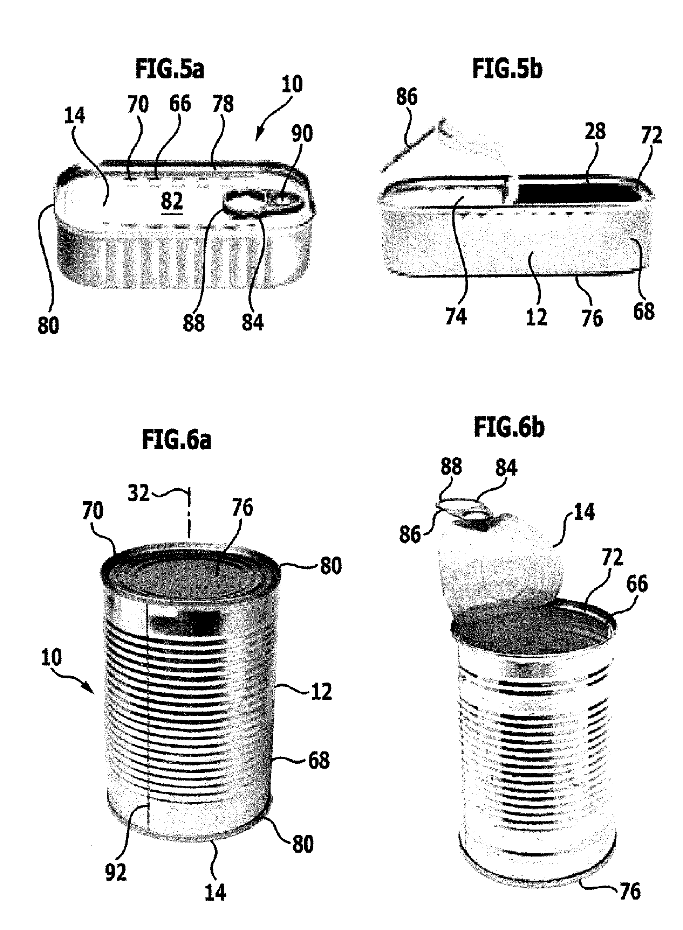

In den Figuren 5a und 5b ist ein fünftes Ausführungsbeispiel einer Sterilverpackung 10 schematisch dargestellt. Es umfasst einen wannenförmigen Behälter 12, welcher eine offene Oberseite 74 aufweist, die durch ein im Wesentlichen plattenförmiges Verschlusselement 14 verschlossen ist. Eine von einem Boden des Behälters 12 in Richtung auf das Verschlusselement 14 abstehende, umlaufende Wand 68 ist in Richtung auf den Aufnahmeraum 28 umgeschlagen und übergreift einen vom Boden 76 weg weisenden, flanschartig umlaufenden Rand 78 des Verschlusselements 14. Durch dieses Umschlagen oder Umbördeln ist das Verschlusselement 14 dauerhaft und gasdicht mit dem Behälter 12 verbunden. Die Umbördelung 80 kann zusätzlich durch Schweißen oder Kleben gesichert werden.

Am Verschlusselement 14 ist ferner eine parallel zum Rand 78 verlaufende, von einer Außenseite 82 des Verschlusselements 14 weg weisende Nut 70 zur Ausbildung einer Sollbruchstelle 66 vorgesehen. Mit dem Verschlusselement 14 ist ein Werkzeugelement 84 verbunden, und zwar in Form eines Zugglieds 86, welches als Zuglasche 88 ausgebildet ist. Es ist mit einem Niet 90 mit dem Verschlusselement 14 verbunden und/oder optional verschweißt oder verklebt. Zum Öffnen der Sterilverpackung 10 wird die in der Lagerstellung am Verschlusselement 14 anliegende Zuglasche 88 von der Außenseite 82 abgehoben und dann auf diese eine Zugkraft ausgeübt. Das Verschlusselement 14 reißt dann längs der Nut 70 auf, sodass das Verschlusselement 14 mittels des Zugglieds 86 abgezogen werden kann, wodurch eine Entnahmeöffnung 72 ausgebildet wird, um das in der Sterilverpackung 10 enthaltene Implantat 26 zu entnehmen.

In den Figuren 6a und 6b ist schematisch ein sechstes Ausführungsbeispiel einer Sterilverpackung 10 dargestellt, und zwar in Figur 6a in der Lagerstellung. Der Aufbau der Sterilverpackung 10, wie sie beispielhaft in den Figuren 6a und 6b dargestellt ist, entspricht konstruktiv dem Aufbau der in den Figuren 5a und 5b schematisch dargestellten Sterilverpackung 10. Der Unterschied besteht jedoch im Wesentlichen in der Form der Sterilverpackung 10. So umfasst der Behälter 12 eine zylindrische Wand 68, die aus einem zylindrisch gebogenen Blechteil gebildet wird, welches zwei durch eine längs einer parallel zur Längsachse 32 verlaufende Schweißnaht 92 verbundene Kanten aufweist.

Der Boden 76 ist wie das Verschlusselement 14 durch Ausbildung einer Umbördelung 80 mit der Wand 68 verbunden.

Am Verschlusselement 14 ist eine Sollbruchstelle 66 in Form einer ringförmig geschlossenen Nut 70 ausgebildet, längs derer das Verschlusselement 14 mittels des Werkzeugelements 84, das als Zugglied 86 ausgebildet ist, aufgerissen werden kann.

Die Sterilverpackungen 10 gemäß dem fünften und sechsten Ausführungsbeispiel sind vorzugsweise aus einem Metall gebildet, und zwar beispielsweise aus einem nicht rostenden Metallblech oder aus Edelstahl.

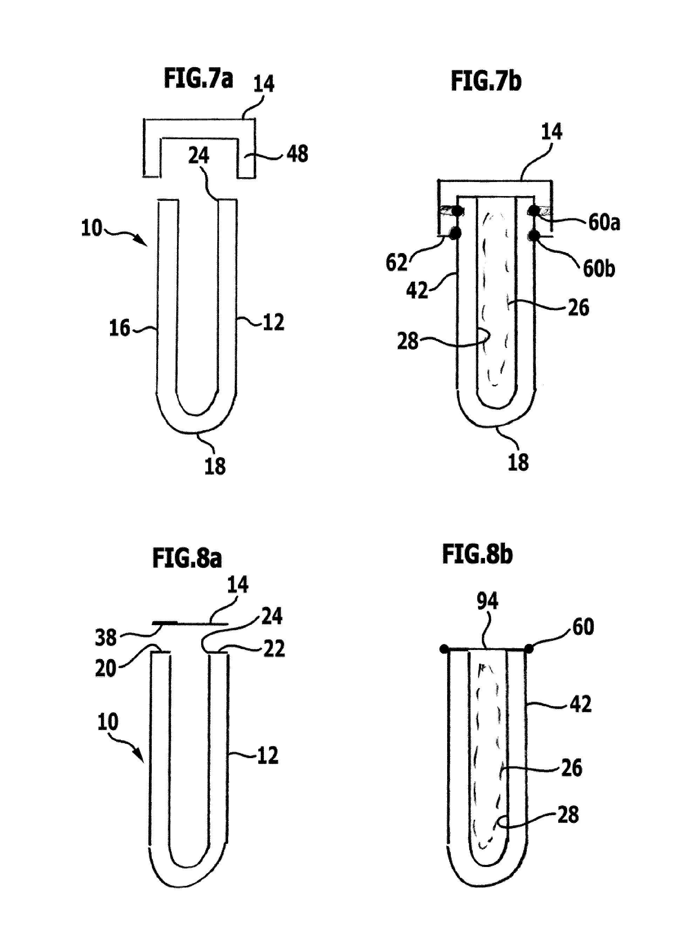

In den Figuren 7a und 7b ist ein siebtes Ausführungsbeispiel einer Sterilverpackung 10 schematisch dargestellt. Der Behälter 12 und das Verschlusselement 14 stimmen im Aufbau im Wesentlichen mit dem zweiten Ausführungsbeispiel der Sterilverpackung 10, die in den Figuren 2a und 2b dargestellt ist, überein und unterscheiden sich von diesem nur dadurch, dass keine Gewindeabschnitte am Behälter 12 und am Verschlusselement 14 vorgesehen sind. Die Befestigung des Verschlusselements 14 am Behälter 12 erfolgt mittels Schweißen, und zwar durch Ausbildung einer ersten Schweißnaht 66a zwischen dem Abschnitt 48 und der Außenseite 42 des Behälters 12, über welchen dieser in der Figur 7b dargestellten Lagerstellung geschoben ist, und einer zweiten Schweißnaht 66b im Übergangsbereich zwischen der in Richtung auf das Ende 18 hin weisenden Ringfläche 62 des Abschnitts 48 und der Außenseite 42. Auf diese Weise kann eine redundante, nämlich doppelte, Abdichtung des Aufnahmeraums 28 ausgebildet werden.

In den Figuren 8a und 8b ist ein achtes Ausführungsbeispiel einer Sterilverpackung 10 schematisch dargestellt. Der Behälter 12 dieses Ausführungsbeispiels entspricht im Wesentlichen dem Behälter 12 des in den Figuren 1a und 1b dargestellten ersten Ausführungsbeispiels der Sterilverpackung 10. Das Verschlusselement 14 wird jedoch durch ein dünnes, kreisscheibenförmiges Blech 94 ausgebildet, dessen Unterseite die Verschlusselementdichtfläche 38 definiert, die in der in Figur 8b dargestellten Lagerstellung an der Randfläche 22 anliegt. Anstatt des Blechs 94 kann eine hinreichend dicke, strapazierfähige Folie genutzt werden, die optional aus mehreren Lagen oder Schichten ausgebildet ist. Die Lagen können aus einem Metall oder einem Kunststoff hergestellt sein, wobei beliebige Kombinationen unterschiedlicher Materialien möglich sind.

Nach dem Einbringen des Implantats 26 in den Aufnahmeraum 28 wird das Verschlusselement 14 auf das Ende 20 aufgesetzt und mit dem Behälter 12 verschweißt, sodass eine umlaufende, die Außenseite 42 und das Blech 94 verbindende Schweißnaht 60 ausgebildet wird.

Diese Sterilverpackung 10 kann beispielsweise mittels eines herkömmlichen Dosenöffners durch Aufschneiden beziehungsweise Durchtrennen des Blechs 94 eröffnet werden.

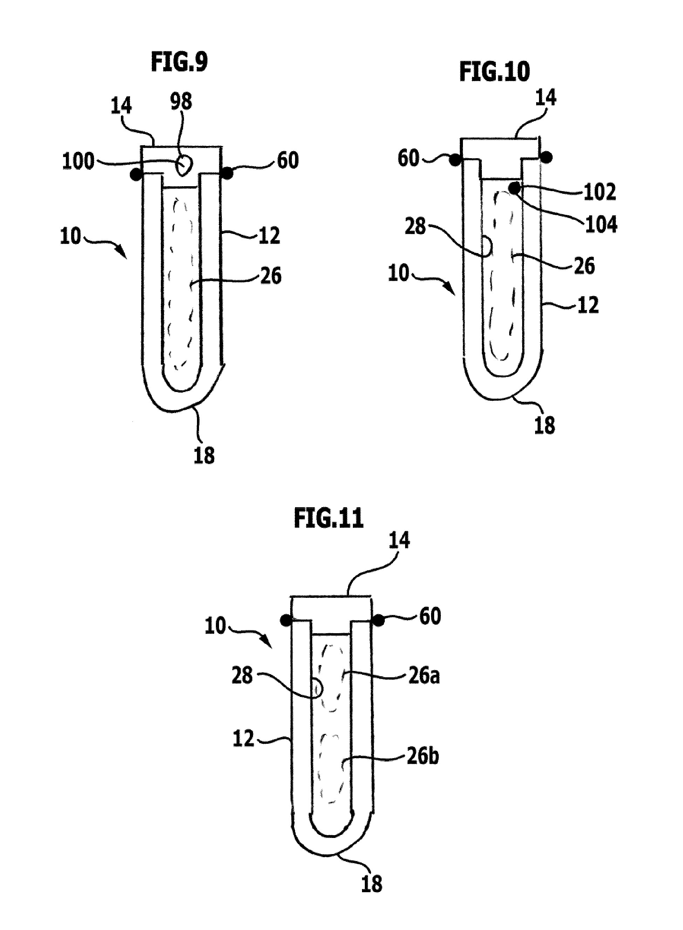

In Figur 9 ist schematisch ein neuntes Ausführungsbeispiel einer Sterilverpackung 10 beispielhaft dargestellt. Es entspricht in seinem Aufbau im Wesentlichen dem in den Figuren 1a und 1b schematisch dargestellten ersten Ausführungsbeispiel der Sterilverpackung 10. Zusätzlich ist an der Sterilverpackung 10 jedoch noch ein Speicherelement 98 angeordnet, und zwar beispielsweise am beziehungsweise im Verschlusselement 14. Bei dem Speicherelement 98 handelt es sich vorzugsweise um einen RFID-Chip 100, welcher in das aus einem Kunststoff ausgebildete Verschlusselement 14 eingegossen sein kann. Im Speicherelement 98 können Informationen zur Charakterisierung des Implantats 26 gespeichert werden, beispielsweise Art und Typ des Implantats 26, Informationen zu dessen Größe sowie Herstellungsdatum, Chargenbezeichnung und/oder eine individuelle Herstellungs- oder Seriennummer.

Das Speicherelement 98 kann wahlweise auch am Behälter 12 angeordnet werden. Optional kann anstatt eines RFID-Chips 100 auch ein in den Figuren nicht dargestellter Barcode genutzt werden, welcher ebenfalls ein berührungsloses Auslesen der gespeicherten Informationen ermöglicht. Der Barcode kann mittels Laserbeschriftung dauerhaft auf die Sterilverpackung aufgebracht sein.

Selbstverständlich können alle oben und auch nachfolgend beschriebenen Ausführungsbeispiele der Sterilverpackung 10 mit einem Speicherelement 98 ausgestattet werden.

Ein zehntes Ausführungsbeispiel einer Sterilverpackung 10 ist schematisch in Figur 10 dargestellt. Es entspricht in seinem Aufbau ebenfalls im Wesentlichen dem in den Figuren 1a und 1b schematisch dargestellten ersten Ausführungsbeispiel der Sterilverpackung 10. Es umfasst jedoch zusätzlich eine Anzeigeeinrichtung 102 zum Anzeigen, ob die Sterilverpackung 10 geöffnet oder beschädigt wurde. Die Anzeigeeinrichtung 102 umfasst vorzugsweise einen Sauerstoffindikator 104, welcher im Aufnahmeraum 28 angeordnet ist. Dieser kann beispielsweise derart ausgebildet sein, dass er beim in Kontakt Treten mit Sauerstoff seine Farbe ändert, sodass beispielsweise dann, wenn der Behälter und/oder das Verschlusselement 14 aus einem transluzenten oder transparenten Material ausgebildet sind, für einen Anwender sofort erkennbar ist, ob Sauerstoff in unerwünschter Weise in den Aufnahmeraum 28 gelangt ist. Wird durch die Anzeigeeinrichtung 102 ein Sauerstoffkontakt angezeigt, ist mit großer Wahrscheinlichkeit davon auszugehen, dass das im Aufnahmeraum 28 verpackte Implantat 26 nicht mehr die für einen chirurgischen Eingriff erforderliche Sterilität aufweist.

Selbstverständlich können auch alle oben und nachfolgend beschriebenen Ausführungsbeispiele der Sterilverpackung 10 optional mit einer Anzeigeeinrichtung 102 ausgestattet werden.

In Figur 11 ist ein elftes Ausführungsbeispiel einer Sterilverpackung 10 schematisch dargestellt. Dieses entspricht in seinem Aufbau dem in den Figuren 1a und 1b dargestellten ersten Ausführungsbeispiel der Sterilverpackung 10. Selbstverständlich ist es möglich, zwei oder mehr Implantate 26a und 26b in den Aufnahmeraum 28 einzubringen und darin zu lagern. Dies ist nicht nur bei dem in Figur 11 dargestellten elften Ausführungsbeispiel möglich, sondern grundsätzlich bei allen oben beschriebenen Ausführungsbeispielen. Um Verwechslungen zu vermeiden, werden vorzugsweise identische Implantate 26a und 26b in den Aufnahmeraum 28 der Sterilverpackung 10 eingebracht.

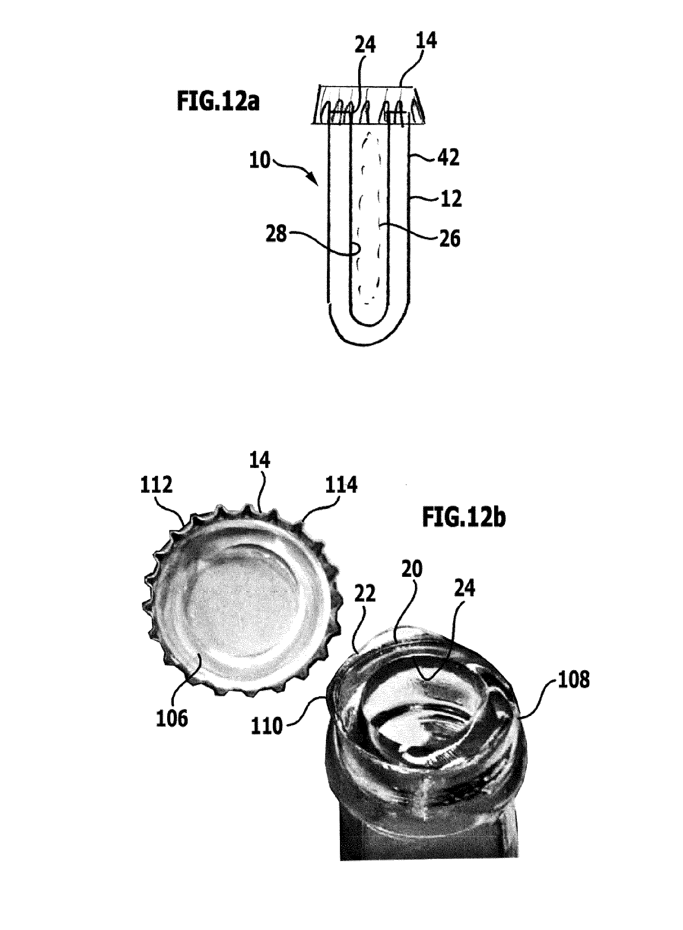

In den Figuren 12a und 12b ist schematisch ein zwölftes Ausführungsbeispiel einer Sterilverpackung 10 dargestellt. Der Behälter 12 entspricht in seinem Aufbau im Wesentlichen dem Behälter 12 des in den Figuren 1a und 1b dargestellten ersten Ausführungsbeispiels der Sterilverpackung 10. Im Bereich des Endes 20 ist vorzugsweise eine von der Außenseite 42 weg weisende Ringnut 108 ausgebildet, sodass am Ende 20 eine wulstartige Verdickung 110 ausgebildet wird. Auf diese ist das in Form eines Kronkorkens 112 ausgebildete Verschlusselement 14 aufgeschoben und durch Ausbildung von mehreren Zacken 114 auf die Verdickung 110 aufgebördelt. Ein Dichtelement 106 ist auf einer Innenseite des Kronkorkens 112 angeordnet, um das Verschlusselement 14 relativ zum Behälter 12 abzudichten. Das Dichtelement 106 liegt in der in Figur 12a schematisch dargestellten Lagerstellung an der Randfläche 22 des Behälters 12.

Der Behälter 12 kann bei diesem Ausführungsbeispiel wahlweise aus einem Metall, einem Kunststoff oder auch aus Glas ausgebildet sein. Das Verschlusselement 14 ist vorzugsweise aus einem Metallblech geformt, das Dichtelement aus einem Kunststoff, vorzugsweise einem elastischen Kunststoff oder zumindest teilelastischen Kunststoff. Möglich sind beispielsweise sterilisierbare Kunststoffe wie Polyethylen, Polyvinylchlorid oder Polyetheretherketon.

Abschließend sei daraufhin gewiesen, dass die in den Figuren schematisch dargestellten und oben beschriebenen Merkmale der erörterten Ausführungsbeispiele der Sterilverpackung 10 grundsätzlich beliebig miteinander kombiniert werden können, das heißt sowohl die Formgebung des Behälters 12 sowie des Verschlusselements 14 als auch die Art, wie diese miteinander verbunden werden. Kraft- oder stoffschlüssige Verbindungen des Behälters 12 mit dem Verschlusselement 14 werden insbesondere durch Verschweißen oder in Abhängigkeit der verwendeten Materialien mit dafür geeigneten Klebestoffe 52 hergestellt.

## Patentansprüche

1. Medizinische Sterilverpackung (10), welche einen Aufnahmeraum (28) für mindestens ein Implantat (26; 26a, 26b), welches ausgebildet ist zum temporären oder dauerhaften Einsetzen in einen menschlichen oder tierischen Körper, definiert, welcher Aufnahmeraum (28) in einer Lagerstellung der Sterilverpackung (10) steril und keimdicht verschlossen ist zum sterilen Verpacken des mindestens einen Implantats (26; 26a, 26b), **dadurch gekennzeichnet, dass** die Sterilverpackung (10) aus einem oder mehreren reinig- und sterilisierbaren Materialien hergestellt ist.

2. Medizinische Sterilverpackung nach Anspruch 1, **dadurch gekennzeichnet, dass** das oder die Materialien lösungsmittelbeständig sind gegen Reinigungs- und Lösungsmittel mit einem pH-Wert in einem Bereich von etwa 2 bis etwa 12, insbesondere in einem Bereich von etwa 3 bis etwa 11.

3. Medizinische Sterilverpackung nach Anspruch 1 oder 2, **dadurch gekennzeichnet, dass** das oder die Materialien wärme-/hitzebeständig sind bis zu einer Temperatur von etwa 150°C, insbesondere bis zu einer Temperatur von etwa 130°C.

4. Medizinische Sterilverpackung nach einem der voranstehenden Ansprüche, **dadurch gekennzeichnet, dass** das oder die Materialien druckbeständig sind bei Drücken bis mindestens etwa 5 bar, insbesondere bei Drücken bis mindestens etwa 3 bar.

5. Medizinische Sterilverpackung nach einem der voranstehenden Ansprüche, **dadurch gekennzeichnet, dass** die Sterilverpackung (10) formstabil ist, insbesondere bei Temperaturen und Drücken in Bereichen gemäß der Ansprüche 3 oder 4.

6. Medizinische Sterilverpackung nach einem der voranstehenden Ansprüche, **dadurch gekennzeichnet, dass** die Sterilverpackung (10) einen Behälter (12) zum Aufnehmen mindestens eines Implantats (26; 26a, 26b) mit einer Einführöffnung (24) und ein Verschlusselement (14) zum Verschließen der Einführöffnung (24) umfasst.

7. Medizinische Sterilverpackung nach einem der voranstehenden Ansprüche, **dadurch gekennzeichnet, dass** die Sterilverpackung (10) mindestens eine Sollbruchstelle (66) zum Öffnen aufweist.

8. Medizinische Sterilverpackung nach Anspruch 6 oder 7, **dadurch gekennzeichnet, dass** der Behälter (12) und das Verschlusselement (14) miteinander verschraubbar ausgebildet sind.

9. Medizinische Sterilverpackung nach einem der Ansprüche 6 bis 8, **dadurch gekennzeichnet, dass** das Verschlusselement (14) einen den Behälter (12) im Bereich der Einführöffnung (24) ringförmig umgebenden Wandabschnitt (48) aufweist.

10. Medizinische Sterilverpackung nach einem der Ansprüche 6 bis 9, **dadurch gekennzeichnet, dass** das Verschlusselement (14) in Form eines Kronkorkens (112) ausgebildet ist.

11. Medizinische Sterilverpackung nach einem der Ansprüche 6 bis 10, **gekennzeichnet durch** ein Dichtelement (106) zum Abdichten des Behälters (12) und des Verschlusselements (14) relativ zueinander.

12. Medizinische Sterilverpackung nach einem der voranstehenden Ansprüche, **gekennzeichnet durch** eine Anzeigeeinrichtung (102) zum Anzeigen, ob die Sterilverpackung (10) geöffnet oder beschädigt wurde.

13. Medizinische Sterilverpackung nach einem der voranstehenden Ansprüche, **gekennzeichnet durch** mindestens ein Speicherelement (98) zum Speichern von Informationen zur Charakterisierung mindestens eines in der Sterilverpackung (10) verpackten Implantats (26; 26a, 26b).

14. Medizinische Sterilverpackung nach einem der voranstehenden Ansprüche, **gekennzeichnet durch** mindestens ein im Aufnahmeraum (28) in der Lagerstellung verpacktes Implantat (26; 26a, 26b).

15. Verwendung einer medizinischen Sterilverpackung (10) nach einem der Ansprüche 1 bis 13 zum sterilen Verpacken mindestens eines Implantats (26; 26a, 26b), welches ausgebildet ist zum temporären oder dauerhaften Einsetzen in einen menschlichen oder tierischen Körper.
